(19) Europäisches Patentamt
European Patent Office
Office européen des brevets

(11) **EP 3 488 238 B1**

(12) **EUROPEAN PATENT SPECIFICATION**

(45) Date of publication and mention of the grant of the patent:
**07.06.2023 Bulletin 2023/23**

(21) Application number: **17743043.6**

(22) Date of filing: **25.07.2017**

(51) International Patent Classification (IPC):
**G01N 33/574** (2006.01)     **G01N 33/68** (2006.01)
**C12Q 1/68** (2018.01)

(52) Cooperative Patent Classification (CPC):
**G01N 33/57419; C12Q 1/6886; G01N 33/6875;**
C12Q 2600/154; C12Q 2600/156

(86) International application number:
**PCT/EP2017/068754**

(87) International publication number:
**WO 2018/019827 (01.02.2018 Gazette 2018/05)**

(54) **COMBINATION TEST FOR COLORECTAL CANCER**

DARMKREBS KOMBINATIONSTEST

TEST COMBINÉ DU CANCER COLORECTAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.07.2016 GB 201612815**

(43) Date of publication of application:
**29.05.2019 Bulletin 2019/22**

(73) Proprietor: **Belgian Volition SRL**
**5032 Isnes (BE)**

(72) Inventors:
• **MICALLEF, Jacob Vincent**
**5032 Isnes (BE)**
• **TERRELL, Jason**
**5032 Isness (BE)**

(74) Representative: **Pond, Elizabeth Grace Catherine**
**Sagittarius IP**
**Marlow International**
**Parkway**
**Marlow SL7 1YL (GB)**

(56) References cited:
**WO-A1-2013/030579     WO-A2-2010/089538**

• **KARL J. ET AL.: "Improved diagnosis of colorectal cancer using a combination of fecal occult blood and novel fecal protein markers", CLIN. GASTROENTEROL. HEPATOL., vol. 6, no. 10, October 2008 (2008-10), pages 1122-1128, XP025518260,**
• **HOLDENRIEDER S. ET AL.: "Novel serum nucleosomics biomarkers for the detection of colorectal cancer", ANTICANCER RES., vol. 34, no. 5, May 2014 (2014-05), pages 2357-2362, XP55119625,**
• **AL-SHUNEIGAT J.M. ET AL.: "Colorectal carcinoma: nucleosomes, carcinoembryonic antigen and ca 19-9 as apoptotic markers; a comparative study", J. BIOMED. SCI., vol. 18, no. 1, 50, 25 July 2011 (2011-07-25), pages 1-5, XP021104561,**
• **PICKHARDT P.J.: "Emerging stool-based and blood-based non-invasive DNA tests for colorectal cancer screening: the importance of cancer prevention in addition to cancer detection", ABDOM. RADIOL., vol. 41, no. 8, 3 June 2016 (2016-06-03), pages 1441-1444, XP036021595,**
• **KIM M. ET AL.: "Fecal occult blood test/fecal carcinoembriogenic antigen dual rapid test as a useful tool for colorectal cancer screening", EUR. SURG., vol. 49, no. 3, 3 February 2017 (2017-02-03), pages 127-131, XP036239392,**

• RAHIER J.-F. ET AL.: "Circulating nucleosomes as new blood-based biomarkers for detection of colorectal cancer", CLIN. EPIGEN., vol. 9, no. 1, 53, 15 May 2017 (2017-05-15), pages 1-7, XP021245140,

**Description**

**FIELD OF THE INVENTION**

The invention relates to detection and screening methods, in particular methods of screening patients for colorectal cancer, a colorectal adenoma or a polyp.

**BACKGROUND OF THE INVENTION**

Colorectal Cancer (CRC) is a common disease with a high mortality. The biology of the disease is understood to involve a progression from pre-cancerous adenoma (polyp) with increasing dysplasia leading to stage I, II, III and eventually stage IV CRC. Mortality varies greatly depending on whether the disease is detected at an early localized stage, when effective treatment options are available, or at a late stage when the disease may have spread within the colon or rectum or beyond when treatment is more difficult. The 5-year survival rate is greater than 90% for those in whom the disease is detected at stage I, but only about 10% for those in whom stage IV metastatic disease is detected. For this reason many countries have CRC screening programmes to identify individuals with CRC or precancerous adenomas or polyps. CRC incidence is age and sex dependent; being more common in men than women and more common in elderly people. CRC is rare in people below the age of 50 so it is more useful to screen older people. The actual screening age range tested varies in screening programmes in different countries but is typically of the order of 50-74 years. In future, screening age ranges may be extended both to younger ages, because of increasing incidence among people aged below 50 years, and to older ages because of increasing life expectancy.

CRC screening programmes enable earlier CRC detection than would otherwise be the case, leading to earlier treatment and many years of saved life. Earlier CRC detection also saves money and resources for healthcare providers by reducing the need for expensive late stage cancer drug therapies and hospitalizations. Ideally CRC screening programmes would also detect precancerous colorectal polyps or adenomas which may progress to CRC if left untreated, but can be removed before cancer develops if detected. The prognosis for such patients is good.

The methods commonly used for CRC detection and/or screening all suffer from major drawbacks. The primary CRC screening method employed in the USA is colonoscopy. Colonoscopy essentially involves visually examining the colon using a scope which traverses the descending, transverse and ascending colon to find any cancerous or potentially pre-cancerous lesions. The primary advantage of colonoscopy is its accuracy of detection which is of the order of 95% clinical sensitivity for CRC as well as very high detection rates for precancerous adenomas all with very high clinical specificity (greater than 95%). This accuracy makes colonoscopy the gold standard for CRC detection, especially as low grade lesions can be removed at the time of their detection. However, colonoscopy suffers from a number of limitations as a frontline CRC detection or screening method. Colonoscopy is a highly invasive procedure requiring a surgical admission, the procedure is usually performed under anesthesia, requires preparation of the bowel by the patient in advance, causes injury to the patient in some cases (for example tearing of the bowel) and is expensive (over $1000). CRC is detected in approximately 0.5% of screening colonoscopies so the vast majority of people screened are subjected to a surgical procedure for little benefit. Due to these disadvantages, patient compliance with colonoscopy is low and many people of screening age do not undergo the procedure. For these reasons, colonoscopy is not used as a frontline CRC detection or screening method in most countries of the world.

Some healthcare providers employ a related method called sigmoidoscopy in which a shorter scope is used to examine the descending colon only. Although this method misses two thirds of the colon, it does examine the area where cancers are most commonly observed. The disadvantages of sigmoidoscopy are similar to those of colonoscopy and it is not commonly used as a frontline test for similar reasons. Virtual colonoscopy, or computerized tomography (CT) colonography, is also used. This procedure employs a combination of x-rays and computer technology to create images of the rectum and colon to detect colorectal tumors and polyps.

Classical biomarkers including carcinoembryonic antigen (CEA) have been investigated as possible blood based biomarkers for CRC but their clinical accuracy is too low for routine use and they are better used for patient monitoring. More recently, hypermethylation of specific gene sequences have been investigated for use as diagnostic biomarkers for cancers in blood. For example, a method reported for detection of hypermethylation of the Septin 9 gene by PCR amplification of DNA extracted from plasma was reported to detect 72% of colon cancers with a false positive rate of 10% (Grutzmann et al, 2008). The DNA methylation status of specific genes or loci is usually detected by selective bisulphite deamination of cytosine, but not 5-methylcytosine, to uracil, leading to a primary DNA sequence change that can be detected by sequencing or other means (Allen et al, 2004).

The most commonly used CRC detection and screening methods involve a two stage procedure in which the population of screening age is first screened with a non-invasive frontline fecal test to identify a subgroup of the screening population in whom there is a higher risk of CRC. People who test positive in the fecal test are referred for a follow-up colonoscopy. The result of fecal screening is negative for the majority of people, so the two stage method prevents unnecessary colonoscopies on most people with no lesion. The result of fecal screening is positive for about 5% of people and this group is deemed at higher risk of CRC. Among this higher risk group approximately 5% of people will typically be found to have CRC. Thus, current fecal tests have poor clinical accuracy and there is a need for affordable, more accurate fecal CRC tests.

The principle underlying current fecal tests for CRC is the detection of bleeding into the colon or rectum. In simple terms; when the colon or rectum is partially blocked by an intruding cancerous or precancerous growth, movement of the stool past the blockage is likely to cause injury and bleeding. This bleeding is detected by testing the fecal sample for the presence of haemoglobin. As the degree of bleeding may vary greatly from day to day, the test may need to be performed several times on separate days.

All current fecal CRC tests are designed to detect fecal haemoglobin. The guaiac fecal occult blood test (FOBT or gFOBT) is a chemical test method for haemoglobin in which the patient or operator typically smears a small amount of feces on to an alpha-guaiaconic acid coated paper or other substrate. If blood is present in the feces, addition of hydrogen peroxide to the paper produces a rapid color change through the oxidation of alpha-guaiaconic acid to a blue colored quinone in a reaction catalyzed by heme (a component of haemoglobin). The consumption of meat (and hence heme) as well as some vegetables, that contain other catalyst molecules that behave like heme in the test, can cause false positive results. Similarly some substances, including vitamin C can lead to false negative results so dietary restriction is often advised prior to the test. Guaiac FOBT tests can have high clinical specificity depending on the cut-off used and have 60-70% sensitivity for detection of CRC. Detection of precancerous polyps or adenomas is poor. Chemical FOBT methods were the method of choice in the past and, although still widely used, are being displaced by fecal immunochemical test methods.

Fecal immunochemical test, or FIT, methods (also called iFOBT or FOBTi) are essentially immunoassay tests for human haaemoglobin in fecal samples. FIT methods are less susceptible to false positive and negative results due to interference of dietary factors and can detect smaller amounts of blood in the feces. These tests have similar specificity to gFOBT but detect slightly more clinically relevant cancer lesions. Detection of polyps or adenomas is poor.

Whole population CRC screening programmes for people of screening age are established in many countries using FIT haemoglobin tests and have resulted in a reduction in the mortality caused by CRC. However, as only about 5% of persons found to be positive for fecal haemoglobin on screening are actually found to have CRC on colonoscopy, most colonoscopies performed are, with hindsight, unnecessary. Performing these unnecessary colonoscopies has a number of detrimental consequences for the patient and for healthcare providers including; (i) large numbers of unnecessary invasive medical colonoscopy procedures performed on healthy persons, (ii) large health care expenditure on expensive and unnecessary colonoscopies, (iii) due to historical insufficient investment in colonoscopy infrastructure, the colonoscopy capability of healthcare providers (particularly in European CRC screening programmes) is currently insufficient to meet medical demand or need resulting in an increasing backlog of unperformed colonoscopies and increased waiting times for colonoscopies for FIT positive persons, and (iv) this increased waiting time has resulted in potentially fatal delayed commencement of CRC treatment for those patients who do have CRC.

CRC screening programmes are struggling with this problem. A common solution is to raise the positive/negative cut-off threshold of the FIT test used, for example, from $20\mu g$ haemoglobin per gram of feces ($20\mu g$ Hb/g feces) to $40\mu g/g$ (or the liquid equivalent concentrations if feces is collected diluted in a buffer), or such as a raise from 1 00ng/ml of fecal haemoglobin to 200ng/ml for the Eiken OC-sensor FIT test. This will result in fewer people being sent for colonoscopy but also in a reduction of the number of those people who do have CRC being detected and hence to an increase in CRC mortality. FIT tests are positive for about 70% of CRC cases at current cut-off thresholds (typically 20 $\mu g/g$ feces) and raising the threshold will reduce this figure further (for example, to 60% or to 65% of CRC cases) meaning that more than one third of all CRC cases will remain undetected.

There is therefore a need in the art for methods to reduce the numbers of people referred for unnecessary colonoscopy whilst maintaining the detection of those people who do have CRC. Counter-intuitively the best way to do this may not be to improve the detection of CRC, but to design a test system to identify a subset of persons who, although they tested positively for fecal haemoglobin, do not have CRC and hence do not need to be referred for colonoscopy. Furthermore, methods to identify persons without disease may also counter-intuitively increase the sensitivity of FIT methods to detect more people with CRC by; (i) increasing the detection rate of follow-up colonoscopy and (ii) allowing for the use of more optimal and dynamic FIT cut-off levels to increase detection among patient groups with higher disease incidence. We now report methods with high negative predictive values for CRC to identify such persons. The methods of the invention may be used to divide people who tested positively for fecal haemoglobin into a very low CRC risk group who do not need a colonoscopy and a high CRC risk group who are in need of a colonoscopy or further investigation, thus reducing colonoscopy referrals whilst continuing to detect all or most of the true CRC cases.

## SUMMARY OF THE INVENTION

According to the invention, there is provided a method for assessing the suitability of a patient for a colonoscopy, comprising:

(a) identifying a patient found to be positive for fecal occult blood;

(b) detecting or measuring the level of two or more moieties in a blood, serum or plasma sample obtained from the patient, wherein the moiety is selected from a cell free nucleosome, and/or an epigenetic feature of a cell free nucleosome; and

(c) using the result obtained in (b) as an indicator of the suitability of the patient for colonoscopy.

## BRIEF DESCRIPTION OF THE FIGURES

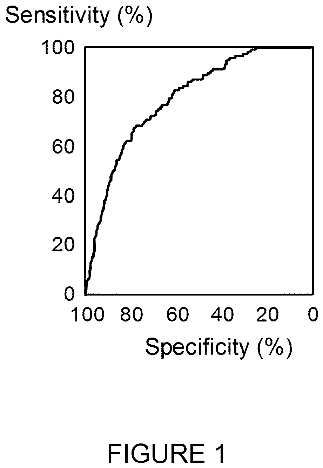

Figure 1. Receiver Operator Curve (ROC) for 1907 FIT positive subjects showing the sensitivity and specificity for CRC detection of a panel including a numerical FIT result as well as ELISA assay results for nucleosomes containing the epigenetic features of 5-methylcytosine, H2AK119Ub, pH2AX, H3K36Me3 and nucleosome-HMGB1 adduct and the subject's age.

## DETAILED DESCRIPTION OF THE INVENTION

The present invention is a FIT combination test performed on persons with a positive fecal haemoglobin test result which may be used to improve the sensitivity and specificity of CRC screening involving such fecal tests, whilst maximizing the best use of colonoscopy resources, improving health economic aspects of screening programs and minimizing the number of people who undergo unnecessary colonoscopy procedures. The method may involve a combination of any two or more of the following parameters;

i. The numerical value of the fecal haemoglobin level measured (FIT level): Higher FIT levels are associated with higher probabilities of CRC;
ii. An epigenetic feature of circulating nucleosomes: Cancer is a disease of genetic and epigenetic mis-regulation and detection of such mis-regulation in circulating serum or plasma nucleosomes and/or their associated DNA has been utilised for cancer detection. We show herein that the establishment of healthy circulating serum/plasma epigenetic nucleosome profiles can be used in combination with other factors to rule out cancer;
iii. A circulating iron metabolism biomarker: In a FIT test the level of haemoglobin above a certain threshold cut-off value is considered a positive result regardless of other factors. However, the presence of haemoglobin in the stool may have been a false positive. Cancer generally may be associated with anemia and, in addition, a true positive fecal haemoglobin result is likely to be associated with gastrointestinal bleeding (GI bleeding) over a protracted time period and to be associated with a disturbance in the patient's iron metabolism. Such a disturbance may be detected by measuring a number of iron metabolism biomarkers in blood/serum/plasma including the patient's haematocrit, packed cell volume, haemoglobin level, ferritin level, transferrin level, soluble transferrin receptor (sTfR) level, hepcidin level, hemojuvelin (HJV) level and/or Bone morphogenetic protein 6 (BMP6) level. Iron metabolism markers have different normal levels in men and women so the patient's sex may be used in the interpretation of results. We show herein that ferritin may be used as a serum biomarker, both alone and in combination, for this purpose. Other markers of iron metabolism disruption may be used because ferritin is an acute phase protein which may be elevated due to inflammation. Such markers include haematocrit and haemoglobin levels which are not associated with inflammation. Transferrin is also not associated with inflammation and can be more sensitive to smaller disruptions of iron metabolism than haematocrit or haemoglobin levels. STfR may also accurately represent a small loss of iron balance and hence be used as a predictor of iron balance health/status. Chronic low level iron metabolism disruption leads to elevated soluble HJV levels which can be detected. Hepcidin is a regulator of systemic iron homeostasis that responds directly to fluctuations in iron levels, and thus can also be used as a marker. If iron levels drop hepatocytes cease hepcidin production, triggering upregulation of dietary iron absorption and systemic mobilization/recycling/conservation of iron. Hepcidin dependent upregulation can maintain neutral iron balance for long periods in chronic low volume GI bleeding. BMP6 levels are also directly related to fluctuations in iron levels. BMP6 is a cytokine active in the main pathway of hepcidin activation.
iv. A Hypoxia Inducible Factor (HIF): Hypoxia inducible factors (HIF) including HIF-1 as well as its subunits HIF-1a, HIF-1b, HIF-2 as well as its subunits HIF-2a and HIF-1b, and HIF-3 are transcription factors induced in hypoxic conditions. They promote a wide range of intra and extracellular changes by altering gene expression allowing cells to adapt and survive in a hypoxic environment due to either or both of systemic anemia, in which all cells of the body experience hypoxia and contribute to elevated HIFs, and hypoxia limited to the tumor microenvironment resulting in a local elevation in HIF synthesis.

v. The level of circulating carcinoembryonic antigen (CEA), or other known markers for CRC: Increasing CEA levels are associated with higher probabilities of CRC disease and with later stage disease.

vi. Other patient parameters, particularly the sex and age, or date of birth, of the patient: Patient parameters such as age, sex, smoking history, weight or Body Mass Index (BMI) are also associated with the probability of CRC disease.

We show herein that combined age and numerical FIT level increases the accuracy of the combined test over use of FIT alone.

Within the disclosure, but not part of the invention, there is described a method of detecting and/or screening for colorectal cancer (CRC), a colorectal adenoma or a polyp in a patient, comprising:

(a) identifying a patient found to be positive for fecal occult blood; and
(b) detecting or measuring the level of one or more moieties in a blood, serum or plasma sample obtained from the patient, wherein the moiety is selected from a cell free nucleosome, an epigenetic feature of a cell free nucleosome, carcinoembryonic antigen, an iron metabolism biomarker or a hypoxia inducible factor,

wherein the result obtained in step (b) is indicative of the likelihood of a patient having colorectal cancer (CRC), a colorectal adenoma or a polyp.

In one embodiment, the patient is identified as having a high likelihood of having colorectal cancer (CRC), a colorectal adenoma or a polyp if the result obtained in step (b) is positive for the patient having CRC, a colorectal adenoma or a polyp. In an alternative embodiment, the patient is identified as having a low likelihood of having colorectal cancer (CRC), a colorectal adenoma or a polyp if the result obtained in step (b) is negative for the patient having CRC, a colorectal adenoma or a polyp.

It will be understood that a patient is deemed to have been tested "positive" in step (a) when fecal occult blood is determined to be present in the fecal sample at a level above the (single) threshold cut-off level used for the test.

The result obtained in step (b) may be used in combination with a numerical fecal occult blood result and/or other factors. In preferred embodiments, a factor selected from one of the 6 groups of factors listed above is used. In highly preferred embodiments, the other factors are patient age and/or sex and/or an iron metabolism biomarker.

Within the disclosure, but not part of the invention, there is further described a method of detecting and/or screening for colorectal cancer (CRC), a colorectal adenoma or a polyp in a patient, comprising:

(a) identifying a patient found to be positive for fecal occult blood; and
(b) detecting or measuring the level of nucleosomes *per se* and/or the level of an epigenetic feature of a cell-free nucleosome in a blood, serum or plasma sample obtained from the patient,

wherein the result obtained in step (b) is indicative of the likelihood of the patient having colorectal cancer (CRC), a colorectal adenoma or a polyp.

It will be appreciated that the term cell-free nucleosome throughout this document is intended to include any cell-free chromatin fragment that includes one or more nucleosomes. Epigenetic features of a cell-free nucleosome as referred to in step (b) may comprise, without limitation, one or more histone post-translational modifications, histone isoforms, modified nucleotides and/or proteins bound to a nucleosome in a nucleosome-protein adduct.

Current fecal occult blood tests, such as the FIT test, are not alone sufficiently accurate enough to successfully diagnose a patient with colorectal cancer, a colorectal adenoma or a polyp. Diagnosis must therefore be confirmed by invasive colonoscopy. However, the present inventors have found that combining numerical fecal occult blood test results with other patient parameters and/or ferritin assays and/or cell free nucleosome assays, provides a test for identifying a subset of patients with no colorectal cancer, or a colorectal adenoma or a polyp.

Out of the patients who are referred for a colonoscopy following a positive fecal occult blood test, only about 5% of these patients will typically be found to have colorectal cancer (CRC). The present inventors have found that combining the fecal occult blood test result with a blood/serum/plasma test and/or with a patient's clinical data, that in combination has a high negative predictive value, provides a combination test which reduces the total number of patients suspected of CRC by approximately 20% to 50% or even more. This reduces the number of healthy patients referred for colonoscopies (for example, by 50%), which not only prevents patients from being subjected to an unnecessary invasive procedure, it also reduces health care costs by reducing the number of colonoscopy referrals.

In a test population who have tested positive in a fecal occult blood test; an ideal test of the invention would allow for the identification of a subgroup of patients, none of whom have CRC. The present inventors have achieved this as shown by the Receiver Operator Curve (ROC) in Figure 1 generated by testing 1907 persons who had tested positive in a fecal occult blood test, using a method of the invention. The ROC curve shows that at a specificity of 25% the sensitivity of the panel test is 100%. This means that these 25% of subjects do not need a colonoscopy and that not subjecting these subjects to further investigation will not result in any missed CRC cases. Thus these 25% of subjects represent a very low CRC risk subgroup among FIT positive group of 1907 subjects. Moreover, the specificity can be increased to 33% whilst maintaining a sensitivity of 97.4%. Thus, using this embodiment of the invention involving a combination of FIT level, age and the levels of 5 circulating serum nucleosome types containing 5-methylcytosine, H2AK119Ub, pH2AX, H3K36Me3 and nucleosome-HMGB1 adduct, referrals for colonoscopy can be reduced by one quarter without missing any CRC cases or reduced by a third whilst missing 2.6% of CRC cases. This compares with a loss of up to 10% of CRC cases by increasing FIT/FOBT test threshold cut-off levels.

Furthermore, the current capabilities for colonoscopy are insufficient to meet screening program needs from patients testing positive for fecal occult blood. Therefore, reducing referrals will be of great operational benefit to healthcare providers, whilst also ensuring that those with the highest likelihood of having CRC will get colonoscopy treatment sooner by reducing waiting times.

Therefore, according to the invention, there is provided a method for assessing the suitability of a patient for a colonoscopy, comprising:

(a) identifying a patient found to be positive for fecal occult blood;
(b) detecting or measuring the level of two or more moieties in a blood, serum or plasma sample obtained from the patient, wherein the moiety is selected from a cell free nucleosome and/or an epigenetic feature of a cell free nucleosome; and
(c) using the result obtained in (b) as an indicator of the suitability of the patient for colonoscopy.

According to a further aspect of the invention, there is provided a method for allocating a subject found to be positive for fecal occult blood in a fecal sample, as having a very low risk/likelihood for CRC or a polyp, or a high risk/likelihood for CRC or a polyp, and hence assessing the suitability of a patient for a colonoscopy, comprising:

(a) testing for fecal occult blood in a fecal sample obtained from the patient;
(b) detecting or measuring the level of nucleosomes *per se* and the level of an epigenetic feature of a cell-free nucleosome in a blood, serum or plasma sample obtained from the patient; and
(c) using the result generated in step (b) as an indicator of the suitability of the patient for colonoscopy.

It will be understood that step (b) is performed when the patient is identified to be positive for fecal occult blood. Furthermore, the result generated in step (b) may be used in combination with a numerical FIT level or other factor(s) when establishing the suitability of a patient for colonoscopy in step (c).

In one embodiment, the result obtained in step (b) is used in combination with a numerical fecal occult blood result, such as a numerical fecal occult blood result obtained in step (a). In a further embodiment, the cut-off level for the numerical fecal occult blood result is at least 10 $\mu$g Hb/g, such as at least 20, 30, 40 or 50 $\mu$g Hb/g, in particular at least about 20$\mu$g Hb/g.

In this aspect, for example, if the result generated in step (b) is positive, then the patient is suitable for colonoscopy. This is because the results obtained in the fecal occult blood test and the high negative predictive value test (e.g. the cell free nucleosome combination test) are both positive. If, however, the result generated in step (b) is negative, then the patient is not suitable for colonoscopy because the high negative predictive value test (e.g. the cell free nucleosome combination test) does not confer with the results of the fecal occult blood test.

Within the disclosure, but not part of the invention, there is further described a method for allocating a subject found positive for fecal occult blood in a fecal sample, as having a very low risk/likelihood for CRC or a polyp, or a high risk/likelihood for CRC or a polyp, comprising:

(a) testing for fecal occult blood in a fecal sample obtained from the patient;
(b) detecting or measuring the level of nucleosomes *per se* and/or the level of an epigenetic feature of a cell-free nucleosome in a blood, serum or plasma sample obtained from the patient; and
(c) using the cell free-nucleosome result generated in (b), optionally in combination with a FIT level and other factor(s), as an indicator of the patient's relative risk of having CRC and/or a polyp.

It will be clear that the object in all of these methods is to identify sub-groups of subjects who either do, or do not, need further investigation for CRC and/or colorectal polyps (for example by colonoscopy or sigmoidoscopy): (i) by identifying individuals who have very low risk of CRC and/or colorectal adenomas and/or colorectal polyps and who do not need further investigation; and/or (ii) by identifying individuals who have a higher risk of CRC and/or colorectal polyps and who do need further investigation.

It will be understood that a patient is deemed to have been tested "positive" in a fecal occult blood test when fecal occult blood is determined to be present in the fecal sample.

The nucleosome assays described herein use the presence or level of nucleosomes *per se* and/or nucleosomes containing particular epigenetic structures as an indicator of the likelihood of said subject having cancer. Therefore, a patient is deemed to have been tested "negative" for suitability for a colonoscopy in step (c) when the level of nucleosomes *per se* and/or presence or level of an epigenetic feature of a cell-free nucleosome is different to one or more "cancer" ("true CRC") control samples, *i.e.* the patient is positive for the presence fecal haemoglobin but is negative for colorectal cancer, a colorectal adenoma or polyp and is not a suitable candidate for colonoscopy.

Within the disclosure, but not part of the invention, there is further described a method of detecting and/or screening for colorectal cancer (CRC), a colorectal adenoma or a polyp in a patient, comprising:

(a) identifying a patient found to be positive for fecal occult blood; and
(b) detecting or measuring the level of an iron metabolism biomarker in a blood, serum or plasma sample obtained from the patient; and
(c) using the iron metabolism biomarker result obtained in step (b), optionally in combination with FIT level and/or other factor(s), as indicative of the likelihood of the patient having colorectal cancer (CRC), a colorectal adenoma or a polyp.

In one embodiment, the iron metabolism biomarker is selected from: ferritin, haematocrit, haemoglobin, transferrin, soluble transferrin receptor (sTfR), hepcidin, hemojuvelin (HJV) and Bone morphogenetic protein 6 (BMP6). In one embodiment, the iron metabolism biomarker is ferritin. In one embodiment, the packed cell volume can also be used as an iron metabolism biomarker.

In one embodiment, the patient is identified as having a high likelihood of having colorectal cancer (CRC), a colorectal adenoma or a polyp if the result obtained in step (c) is positive for the patient having CRC, a colorectal adenoma or a polyp. In an alternative embodiment, the patient is identified as having a low likelihood of having colorectal cancer (CRC), a colorectal adenoma or a polyp if the result obtained in step (c) is negative for the patient having CRC, a colorectal adenoma or a polyp.

It will be understood that a patient is deemed to have been tested "positive" in step (a) when fecal occult blood is determined to be present in the fecal sample at a level above the threshold cut-off level used for the test.

In a population who have tested positive in a fecal occult blood test; an assay of the invention would allow for the identification of a subgroup of patients who are low risk for CRC. Such an assay, in combination with other factors, will contribute to a combination test of the invention. We assayed 599 subjects of screening age tested positive for FIT at a 20μg Hb/g feces cut-off threshold for the iron metabolism marker ferritin. Using a single ferritin level cut-off of 192 ng/ml for both men and women identified a low risk subgroup of 25% of subjects that included only 10% (12 of 118) of CRC cases. Using separate cut-offs for men (208 ng/ml) and women (164 ng/ml) increased the CRC cases missed by 1 case (1% of 118), but decreased the potentially precancerous adenomas missed by 7 cases (4% of 178 cases).

In one embodiment, the iron metabolism biomarker is ferritin and a cut-off level of at least 150 ng/ml, such as at least 160, 170, 180, 190, 200, 210, 220, 230, 240 or 250 ng/ml, in particular 190 ng/ml, is used. The cut-off level can be further distinguished by sex, therefore in a further embodiment, the ferritin cut-off level is at least 190, 195, 200, 205 or 210 ng/ml if the patient is male, and/or the ferritin cut-off level is at least 150, 155, 160, 165, 170, 175, 180, 185 or 190 ng/ml if the patient is female. In a yet further embodiment, the ferritin cut-off level is at least 200 ng/ml if the patient is male, and/or the ferritin cut-off level is at least 160 ng/ml if the patient is female.

In one embodiment, the iron metabolism biomarker result obtained in step (b) is used in combination with a numerical fecal occult blood result, such as a numerical fecal occult blood result obtained in step (a). In a further embodiment, the cut-off level for the numerical fecal occult blood result is at least 10 μg Hb/g, such as at least 20, 30, 40 or 50 μg Hb/g, in particular at least about 20μg Hb/g.

Within the disclosure, but not part of the invention, there is further described a method for allocating a subject found to be positive for fecal occult blood in a fecal sample, as having a very low risk/likelihood for CRC or a polyp, or a high risk/likelihood for CRC or a polyp, and hence assessing the suitability of a patient for a colonoscopy, comprising:

(a) testing for fecal occult blood in a fecal sample obtained from the patient;
(b) detecting or measuring the level of an iron metabolism biomarker in a blood, serum or plasma sample obtained from the patient; and
(c) using the iron metabolism biomarker result generated in step (b), optionally in combination with FIT level and/or other factor(s), as an indicator of the suitability of the patient for colonoscopy.

Within the disclosure, but not part of the invention, there is further described a method for allocating a subject found positive for fecal occult blood in a fecal sample, as having a very low risk/likelihood for CRC or a polyp, or a high risk/likelihood for CRC or a polyp, comprising:

(a) testing for fecal occult blood in a fecal sample obtained from the patient;

(b) detecting or measuring the level of an iron metabolism biomarker in a blood, serum or plasma sample obtained from the patient; and

(c) using the iron metabolism biomarker result generated in (b), optionally in combination with FIT level and/or other factor(s), as an indicator of the patient's relative risk of having CRC and/or a polyp.

Within the disclosure, but not part of the invention, there is further described a method of detecting and/or screening for colorectal cancer (CRC), a colorectal adenoma or a polyp in a patient, comprising:

(a) identifying a patient found to be positive for fecal occult blood; and

(b) detecting or measuring the level of a known CRC biomarker in a blood, serum or plasma sample obtained from the patient; and

(c) using the CRC biomarker result obtained in step (b), optionally in combination with FIT level and/or other factor(s), as indicative of the likelihood of the patient having colorectal cancer (CRC), a colorectal adenoma or a polyp.

In one embodiment, the patient is identified as having a high likelihood of having colorectal cancer (CRC), a colorectal adenoma or a polyp if the result obtained in step (c) is positive for the patient having CRC, a colorectal adenoma or a polyp. In an alternative embodiment, the patient is identified as having a low likelihood of having colorectal cancer (CRC), a colorectal adenoma or a polyp if the result obtained in step (c) is negative for the patient having CRC, a colorectal adenoma or a polyp.

It will be understood that a patient is deemed to have been tested "positive" in step (a) when fecal occult blood is determined to be present in the fecal sample at a level above the (single) threshold cut-off level used for the test.

There are a number of known blood biomarkers for CRC including without limitation carcinoembryonic antigen (CEA), osteopontin, CYFRA-21-1, seprase, Timp-1, anti-p53 antibodies and anti-AFP antibodies. Therefore, in one embodiment, the CRC biomarker is selected from: CEA, osteopontin, CYFRA-21-1, seprase, Timp-1, anti-p53 antibody and anti-AFP antibody. In a further embodiment, the CRC biomarker is CEA.

In a population who have tested positive in a fecal occult blood test; an assay of the invention would allow for the identification of a subgroup of patients who are low risk for CRC. Such an assay, in combination with other factors, will contribute to a combination test of the invention. We assayed 599 subjects of screening age tested positive for FIT at a 20µg Hb/g feces cut-off threshold for the CRC biomarker CEA. Using a single CEA level cut-off of 1.3 ng/ml identified a low risk subgroup of 25% of subjects that included only 15% (18 of 118) of CRC cases.

In one embodiment, the CRC biomarker is CEA and a cut-off level of at least 0.5 ng/ml, such as at least 0.6. 0.7, 0.8, 0.9, 1.0, 1.1, 1.2, 1.3, 1.4 or 1.5 ng/ml, in particular 1.3 ng/ml, is used.

In one embodiment, the CRC biomarker result obtained in step (b) is used in combination with a numerical fecal occult blood result, such as a numerical fecal occult blood result obtained in step (a). In a further embodiment, the cut-off level for the numerical fecal occult blood result is at least 10 µg Hb/g, such as at least 20, 30, 40 or 50 µg Hb/g, in particular at least about 20µg Hb/g.

Within the disclosure, but not part of the invention, there is further described a method for allocating a subject found to be positive for fecal occult blood in a fecal sample, as having a very low risk/likelihood for CRC or a polyp, or a high risk/likelihood for CRC or a polyp, and hence assessing the suitability of a patient for a colonoscopy, comprising:

(a) testing for fecal occult blood in a fecal sample obtained from the patient;

(b) detecting or measuring the level of a known CRC biomarker in a blood, serum or plasma sample obtained from the patient; and

(c) using the CRC biomarker result generated in step (b), optionally in combination with FIT level and/or other factor(s), as an indicator of the suitability of the patient for colonoscopy.

Within the disclosure, but not part of the invention, there is further described a method for allocating a subject found positive for fecal occult blood in a fecal sample, as having a very low risk/likelihood for CRC or a polyp, or a high risk/likelihood for CRC or a polyp, comprising:

(a) testing for fecal occult blood in a fecal sample obtained from the patient;

(b) detecting or measuring the level of a known CRC biomarker in a blood, serum or plasma sample obtained from the patient; and

(c) using the CRC biomarker result generated in (b), optionally in combination with FIT level and/or other factor(s), as an indicator of the patient's relative risk of having CRC and/or a polyp.

Within the disclosure, but not part of the invention, there is further described a method of detecting and/or screening for colorectal cancer (CRC), a colorectal adenoma or a polyp in a patient, comprising:

> (a) identifying a patient found to be positive for fecal occult blood; and
> (b) detecting or measuring the level of a hypoxia inducible factor in a blood, serum or plasma sample obtained from the patient; and
> (c) using the hypoxia inducible factor result obtained in step (b), optionally in combination with FIT level and/or other factor(s), as indicative of the likelihood of the patient having colorectal cancer (CRC), a colorectal adenoma or a polyp.

In one embodiment, the patient is identified as having a high likelihood of having colorectal cancer (CRC), a colorectal adenoma or a polyp if the result obtained in step (c) is positive for the patient having CRC, a colorectal adenoma or a polyp. In an alternative embodiment, the patient is identified as having a low likelihood of having colorectal cancer (CRC), a colorectal adenoma or a polyp if the result obtained in step (c) is negative for the patient having CRC, a colorectal adenoma or a polyp.

In one embodiment, the hypoxia inducible factor is selected from: HIF-1, HIF-2 and HIF-3.

In one embodiment, the hypoxia inducible factor biomarker result obtained in step (b) is used in combination with a numerical fecal occult blood result, such as a numerical fecal occult blood result obtained in step (a). In a further embodiment, the cut-off level for the numerical fecal occult blood result is at least 10 $\mu$g Hb/g, such as at least 20, 30, 40 or 50 $\mu$g Hb/g, in particular at least about 20$\mu$g Hb/g.

Within the disclosure, but not part of the invention, there is further described a method for allocating a subject found to be positive for fecal occult blood in a fecal sample, as having a very low risk/likelihood for CRC or a polyp, or a high risk/likelihood for CRC or a polyp, and hence assessing the suitability of a patient for a colonoscopy, comprising:

> (a) testing for fecal occult blood in a fecal sample obtained from the patient;
> (b) detecting or measuring the level of hypoxia inducible factor in a blood, serum or plasma sample obtained from the patient; and
> (c) using the hypoxia inducible factor result generated in step (b), optionally in combination with FIT level and/or other factor(s), as an indicator of the suitability of the patient for colonoscopy.

Within the disclosure, but not part of the invention, there is further described a method for allocating a subject found positive for fecal occult blood in a fecal sample, as having a very low risk/likelihood for CRC or a polyp, or a high risk/likelihood for CRC or a polyp, comprising:

> (a) testing for fecal occult blood in a fecal sample obtained from the patient;
> (b) detecting or measuring the level of a hypoxia inducible factor in a blood, serum or plasma sample obtained from the patient; and
> (c) using the hypoxia inducible factor result generated in (b) in combination with FIT level and/or other factor(s) as an indicator of the patient's relative risk of having CRC and/or a polyp.

We show herein that the performance of a combination test exceeds that of any individual test used to identify a subgroup of screened FIT positive subjects at low risk for CEA. We used methods of the invention to identify a subgroup of 150 subjects (25%) at low risk for CRC, among 599 subjects tested positive for FIT at a cut-off threshold of 20$\mu$g Hb/g feces. Of the 599 subjects, 118 subjects were diagnosed with CRC. The low risk group of 150 subjects identified by FIT level alone included 6 cases of CRC indicating that simply raising the cut-off level to exclude 25% of colonoscopies would lead to a missing 6 of 118 CRC cases. The low risk groups of 150 subjects identified by either age, CEA level or ferritin level individually included 11, 18 or 12 cases of CRC respectively. A combination test including both FIT level and age identified a low risk group of 150 subjects that included only 6 CRC cases. However, a combination test including FIT level, age, ferritin and CEA identified a low risk group of 150 subjects that included only 2 CRC cases. Thus, this embodiment of the invention may be used to reduce colonoscopy referrals by approximately 25% whilst only missing less than 2% of CRC cases. The numbers of precancerous adenomas were also reduced in the combination test.

FIT tests may be used qualitatively (in which the results are either positive or negative) or quantitatively (in which fecal haemoglobin concentration is estimated). Quantitative fecal immunochemical tests provide a numerical fecal haemoglobin level and allow the end user to choose the cut-off fecal haemoglobin concentration that can be used to identify patients for a follow-up colonoscopy.

In a preferred embodiment, the method of determining that a patient does not have a colorectal cancer, or a colorectal adenoma or polyp, additionally involves use of other patient data in an algorithm. This data may include, without limitation, the patient's numerical fecal haemoglobin level found in a FIT or FOBT test (as opposed to a simple positive/negative result), the patient's age, sex, Body Mass Index (BMI), smoking status and dietary habits. The patient data and the assay data for cell-free nucleosomes and/or iron metabolism biomarkers, and/or hypoxia inducible factors and/or other known biomarkers for CRC are analysed using an algorithm whose output is an indication of the presence or absence of CRC or a polyp, or is a probability indicator of the presence or absence of CRC or a polyp, or is an indicator of the suitability of the patient for colonoscopy.

In one embodiment, the patient's clinical data (particularly the age and/or sex of the patient) is used together with the numerical FIT or FOBT result in an algorithm to determine the presence or absence of colorectal cancer (CRC), a colorectal adenoma or a polyp in the subject. This embodiment provides a method of detecting and/or screening for colorectal cancer (CRC), a colorectal adenoma or a polyp in a subject found positive for fecal occult blood in a fecal sample, comprising:

(i) identifying a patient found to be positive for fecal occult blood;
(ii) using the fecal haemoglobin level and other patient data as an indicator of the likelihood of the subject having colorectal cancer (CRC), a colorectal adenoma or a polyp.

In one embodiment, the method of determining that a patient does not have a colorectal cancer, or a colorectal adenoma or polyp, additionally involves use of other assay data in an algorithm. This data may include any test result found relevant, including without limitation, the patient's blood or fecal carcino-embryonic antigen (CEA) level, C-Reactive Protein (CRP) level, ferritin level, blood haemoglobin level, an iron metabolism biomarker level, a hypoxia inducible factor level and/or other known biomarkers for CRC. These test results may be used in addition to, or in place of, the cell-free nucleosome assays described herein.

Within the disclosure, but not part of the invention, there is further described a method for determining whether a patient found positive for fecal occult blood in a fecal sample, is in need of a colonoscopy, comprising:

(a) identifying a subject found to be positive for fecal occult blood; and
(b) using the age and fecal haemoglobin level of the subject to assign the subject as having a high or low likelihood of having a colorectal cancer, a colorectal adenoma or a polyp.

A high likelihood identifies that a patient is in need of a colonoscopy. Therefore, in one embodiment, the method additionally comprises, (c) using the result generated in step (b) as an indicator of the suitability of the patient for colonoscopy.

Step (b) of this aspect of the invention (to use the age and fecal haemoglobin level of the subject to assign the subject as having a high or low likelihood of having a colorectal cancer, a colorectal adenoma or a polyp) may be conducted using a linear or non-linear algorithm. In one embodiment, the fecal haemoglobin (Hb) level, in μg Hb / g feces, and the age of the patient in years are entered into the linear expression:

$$0.0129 \times \text{FIT LEVEL (μg Hb/g feces)} + 0.0688 \times \text{AGE (yrs)}$$

The output value of the expression may be used in many ways according to the desired outcome. In particular, the expression may be used to provide a value which may be compared to a pre-determined cut-off value in order to assign the subject as having a high or low likelihood of having a colorectal cancer, a colorectal adenoma or a polyp. For example, if the desired outcome is to achieve a reduction in colonoscopies of approximately 25%, then a cut-off value of approximately 5 may be used such that if the output value of the expression is more than 5 then the patient is assigned as high risk for CRC and should be referred for colonoscopy. Correspondingly, if the output value of the expression is less than 5 then the patient is assigned as low risk for CRC and need not be referred for colonoscopy. In one embodiment, the cut-off value is about 5.0, such as between 5.0 and 4.5, or between 4.9 and 4.7. In a further embodiment, the cut-off value is about 4.8.

It will be appreciated by those skilled in the art that the units used for the expression may be altered (for example, age may be expressed in days, weeks, months etc., or Hb levels may be expressed as a concentration after solution and/or dilution or in arbitrary units (AU)) and this will alter the terms in the expression without altering the basis of the method.

Using this method in a cohort of 1907 subjects tested as positive in a FIT test using a cut-off threshold value of 20μg Hb/g feces and a cut-off value of 4.8, we were able to achieve a 25% reduction in colonoscopies whilst maintaining a 96.6% rate of referral for patients with CRC for colonoscopies. Similarly, a referral rate of 88.1% was maintained for patients with High Risk Adenomas. Thus, colonoscopies may be reduced by 25% whilst missing only 3.4% of CRC cases and 11.9% of High Risk Adenoma cases.

In one embodiment, an algorithm of the invention is used a *priori* to establish a variable FIT cut-off level for any given age. For example, the expression above can be converted into a table of varying FIT cut-off levels with increasing age as shown in Table 1. In the cohort of 1907 subjects, a variable cut-off below 20μg Hb/g feces was not possible because all 1907 subjects were FIT positive at the $20\mu$g Hb/g feces threshold. Thus, the column in Table 1 with a minimum cut-off of $20\mu$g Hb/g feces was used. Never-the-less, lower minimum thresholds may be used and we have illustrated this, without limitation, with thresholds of 10 and $16\mu$g Hb/g feces in Table 1. It would also be possible to set the lower threshold at zero $\mu$g Hb/g feces.

Similarly, a maximum higher threshold may be used and we have illustrated this, without limitation, with a maximum threshold of $80\mu$g Hb/g feces in Table 1 with a lower threshold of $10\mu$g Hb/g feces in Table 1. Of course, any level of minimum and/or maximum threshold, or none, may be used.

Table 1.

| Age (years) | FIT cut-off with minimum 20 ($\mu$g Hb/g feces) | FIT cut-off with minimum 16 ($\mu$g Hb/g feces) | FIT cut-off with minimum 10 ($\mu$g Hb/g feces) | FIT cut-off with minimum 10 and maximum 80 ($\mu$g Hb/g feces) |
|---|---|---|---|---|
| 50 | 105.3 | 105.3 | 105.3 | 80 |
| 51 | 99.9 | 99.9 | 99.9 | 80 |
| 52 | 94.6 | 94.6 | 94.6 | 80 |
| 53 | 89.3 | 89.3 | 89.3 | 80 |
| 54 | 83.9 | 83.9 | 83.9 | 80 |
| 55 | 78.6 | 78.6 | 78.6 | 78.6 |
| 56 | 73.3 | 73.3 | 73.3 | 73.3 |
| 57 | 67.9 | 67.9 | 67.9 | 67.9 |
| 58 | 62.6 | 62.6 | 62.6 | 62.6 |
| 59 | 57.3 | 57.3 | 57.3 | 57.3 |
| 60 | 51.9 | 51.9 | 51.9 | 51.9 |
| 61 | 46.6 | 46.6 | 46.6 | 46.6 |
| 62 | 41.2 | 41.2 | 41.2 | 41.2 |
| 63 | 35.9 | 35.9 | 35.9 | 35.9 |
| 64 | 30.6 | 30.6 | 30.6 | 30.6 |
| 65 | 25.2 | 25.2 | 25.2 | 25.2 |
| 66 | 20 | 19.9 | 19.9 | 19.9 |
| 67 | 20 | 16 | 14.6 | 14.6 |
| 68 | 20 | 16 | 10 | 10 |
| > 68 | 20 | 16 | 10 | 10 |

We have shown that use of the variable FIT cut-off levels shown in Table 1 with a lower cut-off level of $20\mu$g Hb/g feces leads to a 25% reduction in colonoscopy referrals, whilst maintaining referral rates of 96.6% and 88.1% of patients with CRC and High Risk Adenomas, respectively.

In current clinical practice, the $20\mu$g Hb/g feces cut-off threshold for FIT testing is the most commonly used threshold and is recommended by manufacturers. This threshold gives an acceptable accuracy of 70-75% sensitivity at a specificity of approximately 95% and represents a compromise to the trade-off between the number of CRC cases missed against the number of unnecessary colonoscopies performed on subjects with no lesion. The trade-off leaves approximately 25-30% of CRC cases as undetected with a colonoscopy referral rate of about 5%. Of the subjects referred for colonoscopy about 5% will be diagnosed with CRC (more than 1% of those screened) and another approximately 30% will be diagnosed with a potentially precancerous adenoma (polyp). Thus, a majority of colonoscopies are performed on subjects who derive no benefit. Using the dynamic cut-off threshold above leads to a decrease in the number of colonoscopy referrals by 25%, whilst increasing the proportion of colonoscopies where a CRC case is discovered to almost 7% and increasing the proportion of colonoscopies where a potentially precancerous adenoma case is discovered to 34%.

Where CRC screening programs have found the trade-off implicit in the $20\mu$g Hb/g feces cut-off used unsuitable, they have invariably either: (i) increased the cut-off to reduce the colonoscopy referral rate and accepted the concomitant increase in the number of cancers missed; or (ii) lowered the cut-off to reduce the number of cancers missed and accepted the concomitant increase in the colonoscopy referral rate. The $20\mu g$ Hb/g feces cut-off threshold for FIT testing is a simple, constant threshold which is used regardless of any other patient parameters. Similarly, other raised or lowered thresholds are simple constant thresholds. However, the pattern of CRC incidence and numerical FIT results is not constant with other patient parameters such as age and sex. In effect what is achieved, using the variable or dynamic cut-off of the present invention, is to exclude colonoscopies among younger patients with relatively low (but greater than $20\mu g$ Hb/g feces) numerical FIT results who are at lower risk for CRC whilst continuing to refer older FIT positive subjects, at higher risk of CRC, for colonoscopy.

Furthermore, although the 1907 subjects referred to above all had a numerical FIT level greater than $20\mu g$ Hb/g feces, it is possible to extend the lower limit of a linear dynamic cut-off to below this level. For example, use of a lower FIT cut-off threshold level less than $20\mu g$ Hb/g feces for subjects at higher ages than 66, in whom the incidence of CRC is higher, would add to the overall sensitivity of the test whilst minimizing the overall colonoscopy rate by avoiding colonoscopies in younger subjects with moderately raised FIT levels with a lower incidence of CRC. Similarly, an upper-limit may be applied as the highest cut-off used for FIT as shown in Table 1.

We investigated a cohort of subjects aged older than 50 years who had been tested as FIT positive at a lower FIT cut-off threshold of $10\mu g$ Hb/g feces including 4 subjects with CRC, 8 subjects with potentially precancerous advanced adenomas and 20 heathy subjects. Among these subjects, 2 of the 4 subjects with cancer, 3 of the 8 subjects with adenomas and 1 of the 20 healthy subjects had FIT values greater than $16\mu g$ Hb/g feces. These data indicate that the method of the invention with a lower cut-off level of $16\mu g$ Hb/g feces, as shown in Table 1, may be used to increase the overall sensitivity of FIT and detect more CRC cases by lowering the minimum lower threshold (for example, to $16\mu g$ Hb/g feces) whilst minimizing the colonoscopy referral rate by offsetting an increase in colonoscopies among older subjects by referral of fewer younger subjects with FIT levels between $20-105\mu g$ Hb/g feces.

Non-linear algorithms may also be used as embodiments of the invention. We have found that non-linear algorithms involving log[FIT] or [FIT]$^{-0.1}$ (FIT level raised to the power of -0.1) are particularly useful embodiments of the invention. In one embodiment, the fecal haemoglobin (Hb) level, in $\mu g$ Hb / g feces, and the age of the patient in years are entered into the expression:

$$0.063 \times AGE\ (yrs) - 19.2 \times (5 \times FIT\ LEVEL)^{-0.1}$$

In another embodiment of the invention, the fecal haemoglobin (Hb) level, in $\mu g$ Hb / g feces, and the age of the patient in years are entered into the log base 2 expression:

$$0.063 \times AGE\ (yrs) - 1.58 \times LOG(FIT\ LEVEL)$$

The output value of these expressions may be used in many ways according to the desired outcome. In particular, the expression may be used to provide a value which may be compared to a pre-determined cut-off value in order to assign the subject as having a high or low likelihood of having a colorectal cancer, a colorectal adenoma or a polyp. For example, if the desired outcome is to achieve a reduction in colonoscopies of approximately 25%, then a cut-off value of approximately 7.4 or 9.5 may be used for the (5 x FIT LEVEL)$^{-0.1}$ or LOG(FIT LEVEL) expressions respectively, such that if the output value of the expression is more than 7.4 or 9.5 respectively, then the patient is assigned as high risk for CRC and should be referred for colonoscopy. Correspondingly, if the output value of the expression is less than 7.4 or 9.5 respectively, then the patient is assigned as low risk for CRC and need not be referred for colonoscopy. It will be clear to those skilled in the art that non-linear expressions can also be transformed into age specific FIT cut-off levels as shown earlier for a linear expression.

When either of these embodiments of the invention was used on a population of 7943 subjects tested positive for FIT at a cut-off of $20\mu g$ Hb/g feces, a low risk group of 25% of subjects was identified which included only 5% of CRC cases (23 of 430 CRC cases).

In one embodiment, the methods described herein, additionally comprise measuring at least one clinical parameter for the patient and using this as a parameter in the interpretation of results. Additional parameters may include any relevant clinical information for example, without limitation, gender, weight, Body Mass Index (BMI), smoking status and dietary habits. Therefore, in a further embodiment, the clinical parameter is selected from: sex and body mass index (BMI).

In one embodiment, the method additionally comprises, treating by colonoscopy and/or surgically and/or administering a therapeutic agent to a subject identified in step (b) as a subject with a high likelihood of having colorectal cancer, a colorectal adenoma or a polyp.

Within the disclosure, but not part of the invention, there is further described a method of treating colorectal cancer (CRC), a colorectal adenoma or a polyp in an animal or human subject comprising:

(a) testing for fecal occult blood in a fecal sample obtained from the patient;
(b) using the age and fecal haemoglobin level of the subject to assign the subject as having a high or low likelihood of having a colorectal cancer, a colorectal adenoma or a polyp; and
(d) treating by colonoscopy and/or surgically and/or administering a therapeutic agent to a subject identified in step (c) as a subject with a high likelihood of having colorectal cancer, a colorectal adenoma or a polyp.

In one embodiment, step (b) comprises using an algorithm as described herein.

In one embodiment, the method additionally comprises, detecting or measuring the level of nucleosomes *per se* and/or the level of an epigenetic feature of a cell-free nucleosome in a blood, serum or plasma sample obtained from the patient.

In one embodiment, the methods described herein comprise detecting or measuring the level of nucleosomes *per se*. It will be appreciated that the level of nucleosomes *per se* may be estimated, for example using immunoassay methods for nucleosomes similar to those known in the art. Enzyme-Linked ImmunoSorbant Assays (ELISA) and several methods have been reported in Salgame et al, 1997; Holdenrieder et al, 2001; and van Nieuwenhuijze et al, 2003. These assays typically employ an anti-histone antibody (for example anti-H2B, anti-H3 or anti-H1, H2A, H2B, H3 and H4) as capture antibody and an anti-DNA or anti-H2A-H2B-DNA complex antibody as detection antibody.

In one embodiment, the cell-free nucleosome is a mononucleosome, oligonucleosome or other chromosome fragment.

The nucleosome is the basic repetitive unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller, 2007).

The structure of nucleosomes in cellular chromatin can vary by Post Translational Modification (PTM) of histone proteins and by the inclusion of variant histone proteins. PTM of histone proteins occurs most commonly, but not only, on the tails of the eight core histones and common modifications include acetylation, methylation or ubiquitination of lysine residues as well as methylation of arginine residues and phosphorylation of serine residues. Histone modifications are known to be involved in epigenetic regulation of gene expression (Herranz and Esteller, 2007). The structure of the nucleosome can also vary by the inclusion of alternative histone isoforms or variants which are different gene or splice products and have different amino acid sequences. Histone variants can be classed into a number of families which are subdivided into individual types. The nucleotide sequences of a large number of histone variants are known and publicly available for example in the National Human Genome Research Institute NHGRI Histone DataBase (http://genome.nhgri.nih.gov/histones/complete.shtml), the GenBank (NIH genetic sequence) DataBase, the EMBL Nucleotide Sequence Database and the DNA Data Bank of Japan (DDBJ).

Histone variant and histone modification patterns present in healthy and diseased cells have been shown to differ in numerous (mostly immunohistochemical) studies (Herranz and Esteller, 2007).

Nucleosome structure also varies by chemical modification of their DNA component, including the methylation status of DNA (Herranz and Esteller, 2007). It has been known in the art for some time that DNA may be methylated at the 5 position of cytosine nucleotides to form 5-methylcytosine and the involvement of DNA methylation in cancer was reported as early as 1983 (Feinberg and Vogelstein, 1983). DNA methylation patterns observed in cancer cells differ from those of healthy cells. Repetitive elements, particularly around pericentromeric areas, are reported to be hypomethylated in cancer relative to healthy cells but promoters of specific genes are reported to be hypermethylated in cancer. The balance of these two effects is reported to result in global DNA hypomethylation in cancer and this is a hallmark of cancer cells (Esteller 2007, Hervouet et al, 2010, Rodriguez-Paredes & Esteller, 2011).

Nucleosome structure also varies by nucleosome binding to any of a multitude of other proteins present in chromatin to form nucleosome adducts. Chromatin comprises a large number of non-histone proteins of a wide variety of types with a variety of functions including transcription factors, transcription enhancement factors, transcription repression factors, histone modifying enzymes, DNA damage repair proteins, nuclear hormone receptors and many more. The study of chromatin bound proteins has been carried out largely by Chromatin ImmunoPrecipitation (ChIP) methods. These methods are well known in the art but are complex, laborious and expensive.

We have previously reported ELISA assays for nucleosomes containing particular epigenetic signals including nucleosomes containing particular histone modifications, particular histone variants and particular DNA modifications as well as particular nucleosome adducts (WO 2005/019826, WO 2013/030579, WO 2013/030577, WO 2013/084002). We have previously used these assays to show that epigenetically altered circulating cell free nucleosomes can be detected in the blood of diseased patients. Therefore, in one embodiment, the detection or measurement (e.g. in step (b)) comprises:

(i) contacting the sample with a first binding agent which binds to cell-free nucleosomes or a component thereof;
(ii) contacting the sample or cell-free nucleosomes with a second binding agent which binds to an epigenetic feature within said cell-free nucleosomes; and
(iii) detecting or quantifying the binding of said second binding agent to the epigenetic feature within said cell-free nucleosomes in the sample.

In an alternative embodiment, the detection or measurement in step (b) comprises:

(i) contacting the sample with a first binding agent which binds to an epigenetic feature within said cell-free nucleosomes;
(ii) contacting the sample or cell-free nucleosomes with a second binding agent which binds to cell-free nucleosomes or a component thereof; and
(iii) detecting or quantifying the binding of said second binding agent to cell-free nucleosomes or a component thereof in the sample.

In one embodiment, the method comprises detecting or measuring an epigenetically altered or otherwise modified cell free nucleosome. Thus, for example, step (b) comprises detecting an epigenetic feature of a cell-free nucleosome.

In one embodiment, the epigenetic feature is selected from: a post-translational histone modification, a histone variant or isoform, a DNA modification or a protein adducted to the nucleosome.

In a further embodiment, the epigenetic feature of a cell-free nucleosome comprises one or more histone post-translational modifications. A large number of such histone modifications are known in the art and this number is increasing with newly identified modifications. For example, without limitation, histones contain a variety of amino acid residues at multiple positions including lysine, serine, arginine and threonine residues in histones H2, H3 and H4, as well as their isoforms or sequence variants. These may be modified in multiple ways including, for example without limitation, acetylation, ubiquitination, biotinylation or mono, di, or tri-methylation of lysine residues. Any histone modification may be a suitable feature for use in the invention whether detected or measured as an individual modified histone moiety, or whether detected or measured as a histone component of a cell free mononucleosome, oligonucleosome or other chromatin fragment, for example, using a chromatographic, mass spectrophotometric, biosensor, chromatin immunoprecipitation (ChIP), immunoassay or other method of detection. In a preferred embodiment nucleosome associated modified histones are measured by means of a 2-site immunoassay (immunometric) method utilising one antibody or other selective binder to a nucleosome epitope and another to the particular histone modification of interest.

In one embodiment of the invention a group or class of related histone modifications (rather than a single modification) is detected. A typical example of this embodiment, without limitation, would involve a 2-site immunoassay employing one antibody or other selective binder directed to bind to nucleosomes and one antibody or other selective binder directed to bind the group of histone modifications in question. Examples of such antibodies directed to bind to a group of histone modifications would include, for illustrative purposes without limitation, anti-pan-acetylation antibodies (e.g. a Pan-acetyl H4 antibody), anti-citrullination antibodies or anti-ubiquitination antibodies.

In one embodiment, the post-translational histone modification is selected from: H2AK119Ub, pH2AX, H3K9Me3, H3K9Ac, H3K27Me3, H3K36Me3, H3S10Ph, H4K16Ac, H4K20Me3, ubiquityl-H2A and H4PanAc (pan-acetylated H4). In a further embodiment, the post-translational histone modification is selected from: H2AK119Ub, pH2AX, H3K36Me3, H3S10Ph and H4PanAc. In a yet further embodiment, the post-translational histone modification is selected from: H2AK119Ub, pH2AX and H3K36Me3.

In an alternative embodiment, the epigenetic feature of a cell-free nucleosome comprises one or more histone variants or isoforms. Many histone variants are known in the art (for example, variants of histone H2 include H2A1, H2A2, mH2A1, mH2A2, H2AX and H2AZ), and this number is increasing with newly identified isoforms. Histone isoform variant protein structures are also amenable to post translational modification. For example, the H2A variant H2AX may be post translationally phosphorylated at serine 139 and this moiety is often called gamma-H2AX. Any histone variant, including any modified variant, may be a suitable feature for use in the invention whether detected or measured as individual histone variant moieties, for example using an immunoassay, chromatographic, mass spectrophotometric, ChIP, biosensor or other method for detection of a histone isoform moiety, or whether detected or measured as a histone component of a cell free mononucleosome, oligonucleosome or other chromatin fragment incorporating the histone variant. In a preferred embodiment nucleosome associated histone variants are measured by means of a 2-site immunoassay utilising one antibody or other selective binder to a nucleosome epitope and another to the particular histone variant or isoform of interest.

In one embodiment, the histone variant or isoform is selected from mH2A1.1, H2AZ and gamma-H2AX.

In an alternative embodiment, the epigenetic feature of a cell-free nucleosome comprises one or more DNA modifications. A number of particular DNA modifications are known in the art (for example, methylation, hydroxymethylation and carboxymethylation of cytosine) and this number is increasing with newly identified modifications. Any modified nucleotide or nucleoside or any DNA modification may be a suitable feature for use in the invention whether detected or measured in isolated DNA, nucleic acid, nucleotide or nucleoside moieties, for example using an immunoassay, chromatographic, mass spectrophotometric, ChIP, biosensor or other method for detection of a modified nucleic acid moiety, or whether detected or measured by any method for cell free mononucleosomes, oligonucleosomes or other chromatin fragments incorporating the particular DNA modification. In a preferred embodiment nucleosome associated DNA modifications are measured by means of a 2-site immunoassay utilising one antibody or other selective binder to a nucleosome epitope and another to the particular DNA modification of interest.

In one embodiment, the DNA modification is selected from 5-methylcytosine or 5-hydroxymethylcytosine.

In a preferred embodiment, the epigenetically modified nucleotide measured in chromatin fragments is 5-methylcytosine or methylated DNA. For clarity this aspect of the invention should not be confused with methods for gene methylation testing which involve the investigation of the cytosine methylation status of a particular gene or DNA sequence, for example as used in the Cologuard method. In contrast the present invention involves determination of the global level of 5-methylcytosine irrespective of gene sequence.

In an alternative embodiment, the epigenetic feature of a cell-free nucleosome comprises one or more protein-nucleosome adducts or complexes. A large number of protein-nucleosome adducts or complexes are known to occur in chromatin (for example,, transcription factors, HMGB1, EZH2 and nuclear hormone receptor-nucleosome adducts) and this number is increasing with newly identified proteins that are associated with chromatin. Any protein-nucleosome adduct may be a suitable feature for use in the invention whether detected or measured by any method for cell free mononucleosome adducts, oligonucleosome adducts or other chromatin fragment adducts incorporating the particular protein, for example a 2-site immunoassay utilising one antibody or other selective binder to a nucleosome epitope and another to the particular protein comprised in the adduct.

In one embodiment, the protein adducted to the nucleosome is selected from: a transcription factor, a High Mobility Group Protein or chromatin modifying enzyme. References to "transcription factor" refer to proteins that bind to DNA and regulate gene expression by promoting (i.e. activators) or suppressing (i.e. repressors) transcription. Transcription factors contain one or more DNA-binding domains (DBDs), which attach to specific sequences of DNA adjacent to the genes that they regulate.

In one embodiment, the nucleosome adduct includes a High Mobility Group Protein, a polycomb protein, a chromatin modifying enzyme or a nuclear hormone receptor.

In one embodiment, the protein adducted to the nucleosome is selected from: HMGB1 and EZH2.

In one embodiment, the detection or measurement in step (b) comprises an immunoassay, immunochemical, mass spectroscopy, chromatographic, chromatin immunoprecipitation or biosensor method.

In one embodiment, the method comprises two or more measurements of cell-free nucleosomes *per se* and/or cell-free nucleosome epigenetic features are performed as a panel of nucleosome features.

In one embodiment, the panel of nucleosome features comprises two or more nucleosome features selected from: 5-methylcytosine, H2AK119Ub, pH2AX, H3K36Me3, H2AZ and HMGB1. In a further embodiment, the panel of nucleosome features comprises: 5-methylcytosine, H2AK119Ub, pH2AX, H3K36Me3, and HMGB1. The panel of nucleosome features may additionally comprise measuring the level of nucleosomes *per se.*

In a further embodiment, the method comprises measuring a panel of nucleosome biomarkers selected from: the level of nucleosomes, one or more particular histone modifications or cell free nucleosomes comprising a particular histone modification, histone variants or cell free nucleosomes comprising a particular histone variant, DNA modifications or cell free nucleosomes comprising a particular DNA modification or nucleosome adducts. As different populations may not be epigenetically identical or similar, the optimum panels selected for different animals or different populations may vary. Thus a panel of such biomarkers selected for use to rule in or to rule out CRC (for example) in humans may not be the same as a panel selected for use in another species (for example cats or dogs). Similarly, the optimal panel for use to rule in or to rule out CRC in male and female humans (or any other animal) may be different. Similarly, the optimal panel for use to rule in or to rule out CRC in different human sub-populations or races may differ. Alternatively, the same panel may be used in different populations (for example in males and females) but with different weightings used for the panel assay members. For example, the test value result of a three assay and patient data parameter panel comprising assays "A", "B" and "C" may be calculated from a mathematical expression such as (Test Value = A + 2B + 3C) in males with a cut-off value of "X" and from a different mathematical expression such as (Test Value = 3A + 2B + C) in females with a different cut-off value of "Y".

In one embodiment, the method for detecting or measuring an epigenetic feature of a cell-free nucleosome in a sample comprises:

(i) obtaining a sample from the subject;

(ii) contacting the sample with a first and second binding agent wherein one of said binding agents binds to cell-free nucleosomes or a component thereof and the other of said binding agents binds to an epigenetic feature of said cell-free nucleosomes; and
(iii) detecting or quantifying the binding of either or both of said binding agents in the sample.

It will be appreciated that the epigenetic features described herein may also be assayed independently of their inclusion, or otherwise, within a nucleosome. Thus, in one embodiment, the method for detecting or measuring an epigenetic feature of a cell-free nucleosome in a sample comprises:

(i) obtaining a sample from the subject; and
(ii) detecting or measuring the level of a particular histone variant, histone isoform, a histone containing a particular post-translational modification or nucleosome-adduct in the sample.

In a preferred embodiment of the invention there is provided a 2-site immunoassay method for the measurement of nucleosome incorporated epigenetic features *in situ* employing an immobilized anti-nucleosome binding agent in combination with a labelled anti-histone modification or anti-histone variant or anti-DNA modification or anti-adducted protein detection binding agent. In another embodiment of the invention there is provided a 2-site immunoassay employing a labelled anti-nucleosome detection binding agent in combination with an immobilized anti-histone modification or anti-histone variant or anti-DNA modification or anti-adducted protein binding agent.

It will be understood that the person identified as positive for fecal occult blood may be identified by: testing for fecal occult blood in a fecal sample obtained from the patient. In one embodiment, the fecal occult blood test is selected from: a Fecal Immunochemical Test (FIT, also known as immunochemical fecal occult blood test or iFOBT), stool guaiac test for fecal occult blood (gFOBT) or Fecal porphyrin quantification (such as HemoQuant). In a further embodiment, the fecal occult blood test is a Fecal Immunochemical Test. FIT products utilize specific antibodies to detect globin and is currently one of the most commonly used colon cancer screening tests.

Devices for the collection of fecal sample material are commercially available, for example in a commercially available FIT test. The device includes a sample stick which is used to scoop a small amount of stool material. The probe is then inserted into its tube, where excess sample is removed from the stick and 10mg of fecal sample is added to 2ml of buffer. The tube also contains a filter system to remove solids when a portion of the liquid sample is removed for analysis. It will be clear to those skilled in the art that any fecal derived sample that has been diluted, filtered, separated, purified or otherwise prepared for analysis may be used be used for the invention.

It will be understood, that although the fecal occult blood test is performed on a fecal sample from the patient, the same or another type of sample may be used in order to test for the one or moieties, such as the level of nucleosomes. In one embodiment, the sample is a body fluid sample, such as a sample selected from: blood or serum or plasma (in particular, a blood sample). It will be clear to those skilled in the art that the detection of nucleosome adducts in a body fluid has the advantage of being a minimally invasive method that does not require biopsy.

In one embodiment, the disease is selected from cancer, such as colorectal cancer, a colorectal adenoma or a polyp.

In addition, further tests may be included with the method of the present invention to increase its clinical accuracy including, for example tests, for haemoglobin, other known tumor markers including carcinoembryonic antigen (CEA) and/or specific mutated or methylated gene sequences. Therefore, in one embodiment, the method additionally comprises detecting or measuring the level of carcinoembryonic antigen (CEA) in a sample obtained from the patient.

In one embodiment, the method additionally comprises testing for specific mutated or methylated gene sequences. For example, there are currently fecal tests for CRC detection which involve analysis of DNA sequences in the stool in addition to analysis for haemoglobin. In these methods DNA is extracted from the stool and analyzed to identify cancer associated sequence mutations and/or gene specific DNA methylation changes. An example of this approach is provided by the Cologuard test produced by Exact Sciences Corporation. The test involves a panel of 11 markers for CRC in the stool including haemoglobin, 7 DNA sequence mutations, 2 DNA sequence methylations and the DNA sequence for beta actin (as a control for normalization of results). In this test, the patient stool sample is processed at the laboratory to isolate the DNA from the stool. The DNA is then amplified and NDRG4 and BMP3 gene methylation is investigated by quantitative allele-specific real-time target and signal amplification. Cancer associated KRAS mutations are also investigated using these assays and all are assessed in relation to the level of total DNA assessed by beta actin amplification. DNA methylation, mutation and haemoglobin results are combined to provide a positive or negative result. The Cologuard test is more accurate than simple FOBT or FIT tests and detects 92% of cancers and 42% of polyps with a specificity of 87% (The Medical Letter, 2014). Combining this test with the nucleosomes assays described for the present invention would further increase the accuracy of these tests.

In one embodiment, the method additionally comprises measuring at least one clinical parameter for the patient. Additional parameters may include any relevant clinical information for example without limitation age, gender, Body Mass Index (BMI), smoking status and dietary habits. Therefore, in a further embodiment, the clinical parameter is selected from: age, sex and body mass index (BMI).

In one embodiment, the one or more binding agents comprises a ligand or binder specific for the cell-free nucleosome or component part thereof, or a structural/shape mimic of the nucleosome or component part thereof.

In one embodiment of the invention, the antibody or other selective binder selected as the anti-nucleosome binder is selective for the enrichment of nucleosomes of tumor origin. In a preferred embodiment the antibody or other anti-nucleosome selective binder used is directed to bind to histone H3.1 and/or H3.2 and/or H3t. The binder selective for nucleosomes of tumor origin may be used in any assay including assays for the level of nucleosomes *per se* (of tumor origin), any particular histone modification or cell free nucleosomes comprising a particular histone modification, histone variant or cell free nucleosomes comprising a particular histone variant, DNA modification or cell free nucleosomes comprising a particular DNA modification or a nucleosome adduct.

Any method for isolating and/or detecting or measuring the level of histone modifications and/or cell free nucleosomes comprising a particular histone modification and/or histone variants and/or cell free nucleosomes comprising a particular histone variant and/or DNA modifications and/or cell free nucleosomes comprising a particular DNA modification and/or nucleosome adducts and/or nucleosomes *per se* as biomarkers in a sample may be used for the invention. Examples of methods for the detection or measurement of theses analytes include chromatographic, spectroscopic methods (particularly mass spectrometry), biosensor, ChIP and immunochemical methods. Some detection or measurement methods may involve prior enrichment or isolation of these analytes from the sample, for example by methods including chromatography or affinity purification/isolation using a selective antibody binder or other specific analyte binders. DNA modification isolation or purification may be achieved by DNA extraction methods known in the art.

It will be clear to those skilled in the art that the terms antibody, binder or ligand in regard to any aspect of the invention is not limiting but intended to include any binder capable of binding to particular molecules or entities and that any suitable binder can be used in the method of the invention. It will also be clear that the term "nucleosomes" is intended to include mononucleosomes and oligonucleosomes and any such chromatin fragments that can be analysed in fluid media.

In one embodiment, the ligands or binders of the invention include naturally occurring or chemically synthesised compounds, capable of specific binding to the desired target. A ligand or binder may comprise a peptide, an antibody or a fragment thereof, or a synthetic ligand such as a plastic antibody, or an aptamer or oligonucleotide, capable of specific binding to the desired target. The antibody can be a monoclonal antibody or a fragment thereof. A ligand may be labeled with a detectable marker, such as a luminescent, fluorescent, enzyme or radioactive marker; alternatively or additionally a ligand according to the invention may be labelled with an affinity tag, *e.g.* a biotin, avidin, streptavidin or His (e.g. hexa-His) tag. Alternatively ligand binding may be determined using a label-free technology for example that of ForteBio Inc.

The term "detecting" or "diagnosing" as used herein encompasses identification, confirmation, and/or characterisation of a disease state. Methods of detecting, monitoring and of diagnosis according to the invention are useful to confirm the existence of a disease, to monitor development of the disease by assessing onset and progression, or to assess amelioration or regression of the disease. Methods of detecting, monitoring and of diagnosis are also useful in methods for assessment of clinical screening, prognosis, choice of therapy, evaluation of therapeutic benefit, *i.e.* for drug screening and drug development.

The immunoassays of the invention include any method employing one or more antibodies or other specific binders directed to bind to a nucleosome or to a nucleosome component or to an epigenetic feature of a nucleosome. Immunoassays include 2-site immunoassays or immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays as well as single-site immunoassays, reagent limited immunoassays, competitive immunoassay methods including labelled antigen and labelled antibody single antibody immunoassay methods with a variety of label types including radioactive, enzyme, fluorescent, time-resolved fluorescent and particulate labels. All of said immunoassay methods are well known in the art, see for example Salgame *et al,* 1997 and van Nieuwenhuijze *et al*, 2003.

Identifying and/or quantifying can be performed by any method suitable to identify the presence and/or amount of a specific protein in a biological sample from a subject or a purification or extract of a biological sample or a dilution thereof. In methods of the invention, quantifying may be performed by measuring the concentration of the target in the sample or samples. Biological samples that may be tested in a method of the invention include those as defined hereinbefore. The samples can be prepared, for example where appropriate diluted or concentrated, and stored in the usual manner.

Identification and/or quantification of biomarkers may be performed by detection of the biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length. It is noted in particular that peptides of the same or related sequence to that of histone tails are particularly useful fragments of histone proteins.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Within the disclosure, but not part of the invention, there is further described a method of treating colorectal cancer (CRC), a colorectal adenoma or a polyp in an animal or human subject comprising:

(a) testing for fecal occult blood in a fecal sample obtained from the patient;
(b) detecting or measuring the level of one or more moieties in a blood, serum or plasma sample obtained from the subject, wherein the moiety is selected from a cell free nucleosome, an epigenetic feature of a cell free nucleosome, carcinoembryonic antigen, an iron metabolism or a hypoxia inducible factor;
(c) using the result generated in (b) as indicative of the likelihood of the subject having colorectal cancer, a colorectal adenoma or a polyp; and
(d) treating by colonoscopy and/or surgically and/or administering a therapeutic agent to a subject identified in step (c) as a subject with a high likelihood of having colorectal cancer, a colorectal adenoma or a polyp.

Within the disclosure, but not part of the invention, there is further described a method of treating colorectal cancer (CRC), a colorectal adenoma or a polyp in an animal or human subject comprising:

(a) testing for fecal occult blood in a fecal sample obtained from the patient;
(b) detecting or measuring the level of nucleosomes *per se* and/or the level of an epigenetic feature of a cell-free nucleosome in a blood, serum or plasma sample obtained from the subject;
(c) using the cell free-nucleosome result generated in (b) as indicative of the likelihood of the subject having colorectal cancer, a colorectal adenoma or a polyp; and
(d) treating by colonoscopy and/or surgically and/or administering a therapeutic agent to a subject identified in step (c) as a subject with a high likelihood of having colorectal cancer, a colorectal adenoma or a polyp.

Within the disclosure, but not part of the invention, there is further described a method of treating colorectal cancer (CRC), a colorectal adenoma or a polyp in a patient in need thereof, which comprises the step of treating by colonoscopy and/or surgically and/or administering a therapeutic agent to a patient: (i) testing positive for fecal occult blood in a fecal sample; and (ii) with differing or similar levels of one or moieties selected from: nucleosomes *per se,* an epigenetic feature of a cell-free nucleosome, carcinoembryonic antigen, an iron metabolism or a hypoxia inducible factor, in a sample when compared to the levels of said moiety from a control subject.

Within the disclosure, but not part of the invention, there is further described a method of treating colorectal cancer (CRC), a colorectal adenoma or a polyp in a patient in need thereof, which comprises the step of treating by colonoscopy and/or surgically and/or administering a therapeutic agent to a patient: (i) testing positive for fecal occult blood in a fecal sample; and (ii) with differing or similar levels of nucleosomes *per se* and/or an epigenetic feature of a cell-free nucleosome in a sample when compared to the levels of nucleosomes and/or said epigenetic feature of a cell-free nucleosome from a control subject.

In one embodiment, the control subject is a healthy/non-diseased subject. Therefore, in this embodiment, it will be understood that a different level of nucleosomes *per se* and/or an epigenetic feature of a cell-free nucleosome in a sample is indicative that the patient has a high likelihood of having colorectal cancer (CRC), a colorectal adenoma or a polyp, and should therefore be treated. In an alternative embodiment, the control subject is a subject diagnosed with CRC (i.e. a "true CRC" subject), a colorectal adenoma or a polyp. In this embodiment, it will be understood that a different level of nucleosomes *per se* and/or an epigenetic feature of a cell-free nucleosome in a sample is indicative that the patient has a low likelihood of having colorectal cancer (CRC), a colorectal adenoma or a polyp, and should therefore not be treated.

There are many methods for treating colorectal cancer, such as by colonoscopy and/or surgically and/or administering a therapeutic agent to a patient. Treatment by surgery may comprise laproscopic surgery or colostomy for rectal cancer. Treatment may also comprise radiation therapy, such as external beam radiation therapy or intraoperative radiation therapy (i.e. given during surgery). In one embodiment, the therapeutic agent may comprise a chemotherapy, for example, a chemotherapy selected from: Capecitabine (Xeloda), Fluorouracil (5-FU, Adrucil), Irinotecan (Camptosar), Oxaliplatin (Eloxatin), Bevacizumab (Avastin), Cetuximab (Erbitux), Panitumumab (Vectibix), Ramucirumab (Cyramza), Regorafenib (Stivarga), Zivaflibercept (Zaltrap), or combinations thereof (e.g. Xelox which is a combination of Oxaliplatin and Capecitabine). Targeted therapies may also be used, such as antiangiogenesis therapy or Epidermal Growth Factor Receptor (EGFR) inhibitors.

Diagnostic kits for the diagnosis and monitoring of the presence of a disease state are described herein, but do not form part of the invention. Therefore, within the disclosure, but not part of the invention, there is further described a kit for use in diagnosing colorectal cancer, a colorectal adenoma or a polyp comprising:

(a) a Fecal Occult Blood Test (FOBT) and/or a Fecal Immunochemical Test (FIT); and
(b) a ligand or binder specific for one or more moieties selected from a cell free nucleosome, an epigenetic feature of a cell free nucleosome, carcinoembryonic antigen, an iron metabolism or a hypoxia inducible factor.

Within the disclosure, but not part of the invention, there is further described a kit for use in diagnosing colorectal cancer, a colorectal adenoma or a polyp comprising:

(a) a Fecal Occult Blood Test (FOBT) and/or a Fecal Immunochemical Test (FIT); and
(b) a ligand or binder specific for detection of epigenetic feature of a cell-free nucleosome and/or cell-free nucleosomes *per se.*

Suitably a kit may contain one or more components selected from the group: a ligand binder, or ligands, specific for the epigenetic feature of a cell-free nucleosome and/or cell-free nucleosomes *per se* or a structural/shape mimic thereof, one or more controls, one or more reagents and one or more consumables; optionally together with instructions for use of the kit in accordance with any of the methods defined herein.

It will be understood that the embodiments described herein may be applied to all aspects of the invention.

The invention will now be illustrated with reference to the following non-limiting examples.

## EXAMPLE 1

1907 persons who tested positive in a FIT (fecal occult blood) test and were for this reason referred for a colonoscopy gave informed consent for giving a blood sample and for anonymous use of patient data. The blood samples were taken prior to colonoscopy and analysed using a number of assays for cell-free nucleosomes including nucleosomes *per se,* and nucleosomes containing the epigenetic features of 5-methylcytosine modified DNA, histone variants H2AZ and mH2A1.1, histone modifications pH2AX, H2AK119Ub, H3K36Me3, H4K20Me3, H4PanAc and H3S10Ph and the nucleosome adducts nucleosome-HMGB1 and nucleosome-EZH2.

All of these nucleosome assays were useful for methods of the invention. The results for one panel involving 5 nucleosome assays (5-methylcytosine, H2AK119Ub, pH2AX, H3K36Me3 and nucleosome-HMGB1 adduct) together with the person's numerical FIT level and age for the identification of CRC cases against cases with no findings on colonoscopy are shown in the ROC curve in Figure 1. The ROC curve shows that at a specificity of 25% the sensitivity of the panel test is 100% (118 of 118 persons diagnosed with CRC were correctly identified as in need of a colonoscopy). This means that these 25% of subjects do not need a colonoscopy and that not subjecting these subjects to further investigation will not result in any missed CRC cases. Thus these 25% of subjects represent a very low CRC risk subgroup among FIT positive group of 1907 subjects which can be identified prior to colonoscopy. Moreover, the specificity can be increased to 33% whilst maintaining a sensitivity of 97.4% (115 of 118 persons diagnosed with CRC were correctly identified as in need of a colonoscopy). Thus, referrals for colonoscopy can be reduced by one quarter without missing any CRC cases or reduced by a third whilst missing 2.6% of CRC cases. This compares with a loss of up to 10% of CRC cases by increasing FIT/FOBT test threshold cut-off levels.

## EXAMPLE 2

1907 persons who tested positive in a FIT (fecal occult blood) test and were for this reason referred for a colonoscopy gave informed consent for anonymous use of patient data. For each person their fecal haemoglobin (Hb) level, in $\mu$g Hb / g feces, and the age of the patient in years were entered into the expression:

$$0.0129 \times \text{FIT LEVEL (µg Hb/g feces)} + 0.0688 \times \text{AGE (yrs)}$$

If the output value of the expression was greater than 4.8 then the patient was assigned as high risk for CRC and in need of a colonoscopy. Correspondingly, if the output value of the expression was less than 4.8 then the patient was assigned as low risk for CRC and not in need of a colonoscopy.

Of 118 diagnosed cases of CRC, 114 were correctly assigned as in need of a colonoscopy using this method.

Similarly, 222 of 252 cases of High Risk Adenoma (88.1%) were correctly assigned as in need of a colonoscopy. The ROC curve shows that at a specificity of 25% the sensitivity of the method for CRC is above 95%. This means that not subjecting these subjects to further investigation would have resulted in fewer than 5% missed CRC cases. Thus, these 25% of subjects represent a low CRC risk subgroup among FIT the positive group of 1907 subjects which were identified prior to colonoscopy. This compares with a loss of up to 10% of CRC cases by increasing FIT/FOBT test threshold cut-off levels.

**EXAMPLE 3**

599 persons who tested positive in a FIT (fecal occult blood) test and were for this reason referred for a colonoscopy gave informed consent for giving a blood sample and for anonymous use of patient data. The blood samples were taken prior to colonoscopy and analysed for ferritin and CEA. The assay results in combination with the patient's age and numerical FIT results were entered into the expression;

$$0.51 \times AGE \text{ (yrs)} + 0.17 \times CEA \text{ (ng/ml)} - 17.85 \times (5 \times FIT)^{-0.1} \text{ (µg Hb/g feces)} - 0.17 \times FERRITIN \text{ (ng/ml)}$$

This expression was set to provide a 25% reduction in colonoscopy referral. If the output value of the expression was greater than -8.6 then the patient was assigned as high risk for CRC and in need of a colonoscopy. Correspondingly, if the output value of the expression was less than -8.6 then the patient was assigned as low risk for CRC and not in need of a colonoscopy. Of 118 diagnosed cases of CRC, 116 were correctly assigned as in need of a colonoscopy using this method of the invention, demonstrating that 25% of colonoscopies may be avoided whilst missing less than 2% of cancer cases. Similarly, 79 of 87 cases of high risk adenomas were correctly assigned as in need of a colonoscopy, demonstrating that 9% of high risk adenoma cases were missed.

**REFERENCES**

Allen et al. (2004) Nucleic Acids Research: 32(3) e38
Esteller, (2007) Nature Reviews Genetics: 8, 286-298
Feinberg and Vogelstein, (1983) Nature: 301, 89-92, 1983
Grutzmann et al. (2008) PLoS ONE 3(11): e3759
Hervouet et al. (2010) PLoS ONE 5(6): e11333
Herranz and Esteller, (2007) Methods Mol. Biol.: 361, 25-62
Holdenrieder et al. (2001) Int. J. Cancer (Pred. Oncol.): 95, 114-120
The Medical Letter on Drugs and Therapeutics (2014) 56, 100-101
Rodriguez-Paredes and Esteller, (2011) Nature Medicine: 17(3), 330-339
Salgame et al. (1997) Nucleic Acids Research: 25(3), 680-681
van Nieuwenhuijze et al. (2003) Ann. Rheum. Dis.: 62: 10-14

**Claims**

1. A method for assessing the suitability of a patient for a colonoscopy, comprising:

   (a) identifying a patient found to be positive for fecal occult blood;
   (b) detecting or measuring the level of two or more moieties in a blood, serum or plasma sample obtained from the patient, wherein the moiety is selected from a cell free nucleosome and/or an epigenetic feature of a cell free nucleosome; and
   (c) using the result obtained in step (b) as an indicator of the suitability of the patient for colonoscopy.

2. The method as defined in claim 1, wherein step (a) comprises testing for fecal occult blood in a fecal sample obtained from the patient.

3. The method as defined in claim 1 or claim 2, which additionally comprises use of a numerical fecal occult blood result as a parameter in step (b).

4. The method as defined in any one of claims 1 to 3, which additionally comprises use of a clinical parameter in step (b).

5. The method as defined in claim 4, wherein the clinical parameter is selected from: age, sex and body mass index (BMI).

6. The method as defined in any one of claims 1 to 5, wherein the epigenetic feature is selected from: a post-translational histone modification, a histone variant or isoform, a DNA modification or a protein adducted to the nucleosome.

7. The method as defined in any one of claims 1 to 6, wherein the method additionally comprises detecting or measuring the level of carcinoembryonic antigen (CEA).

**Patentansprüche**

1. Verfahren zum Beurteilen der Eignung eines Patienten für eine Darmspiegelung, umfassend:

   (a) Identifizieren eines Patienten, der positiv auf okkultes Blut im Stuhl getestet wurde;
   (b) Nachweisen oder Messen des Spiegels von zwei oder mehr Einheiten in einer Blut-, Serum- oder Plasmaprobe, die von dem Patienten erlangt wird, wobei die Einheit aus einem zellfreien Nukleosom und/oder einem epigenetischen Merkmal eines zellfreien Nukleosoms ausgewählt ist; und
   (c) Verwenden des in Schritt (b) erlangten Ergebnisses als ein Indikator für die Eignung des Patienten für eine Darmspiegelung.

2. Verfahren nach Anspruch 1, wobei Schritt (a) Testen auf okkultes Blut im Stuhl in einer von dem Patienten erlangten Stuhlprobe umfasst.

3. Verfahren nach Anspruch 1 oder Anspruch 2, das zusätzlich Verwenden eines numerischen Ergebnisses für okkultes Blut im Stuhl als einen Parameter in Schritt (b) umfasst.

4. Verfahren nach einem der Ansprüche 1 bis 3, das zusätzlich Verwenden eines klinischen Parameters in Schritt (b) umfasst.

5. Verfahren nach Anspruch 4, wobei der klinische Parameter ausgewählt ist aus: Alter, Geschlecht und Body-Mass-Index (BMI) .

6. Verfahren nach einem der Ansprüche 1 bis 5, wobei das epigenetische Merkmal ausgewählt ist aus: einer posttranslationalen Histonmodifikation, einer Histonvariante oder -isoform, einer DNA-Modifikation oder einem an das Nukleosom adduzierten Protein.

7. Verfahren nach einem der Ansprüche 1 bis 6, wobei das Verfahren zusätzlich Nachweisen oder Messen des Spiegels an karzinoembryonalem Antigen (CEA) umfasst.

**Revendications**

1. Procédé d'évaluation de l'aptitude d'un patient à subir une coloscopie, comprenant :

   (a) l'identification d'un patient dont la présence de sang occulte dans les selles est positive ;
   (b) la détection ou la mesure du niveau de deux fractions ou plus dans un échantillon de sang, de sérum ou de plasma prélevé sur le patient, dans lequel la fraction est sélectionnée à partir d'un nucléosome acellulaire et/ou une caractéristique épigénétique d'un nucléosome acellulaire ; et
   (c) l'utilisation du résultat obtenu à l'étape (b) comme indicateur de l'aptitude du patient à la coloscopie.

2. Procédé selon la revendication 1, dans lequel l'étape (a) comprend le test de sang occulte dans les selles dans un échantillon fécal obtenu du patient.

3. Procédé selon la revendication 1 ou la revendication 2, qui comprend en outre l'utilisation d'un résultat numérique de sang occulte dans les selles comme paramètre à l'étape (b).

**4.** Procédé selon l'une quelconque des revendications 1 à 3, qui comprend en outre l'utilisation d'un paramètre clinique à l'étape (b).

**5.** Procédé selon la revendication 4, dans lequel le paramètre clinique est choisi parmi : l'âge, le sexe et l'indice de masse corporelle (IMC).

**6.** Procédé selon l'une quelconque des revendications 1 à 5, dans lequel la caractéristique épigénétique est choisie parmi : une modification d'histone post-traductionnelle, un variant ou isoforme d'histone, une modification d'ADN ou une protéine ajoutée au nucléosome.

**7.** Procédé selon l'une quelconque des revendications 1 à 6, dans lequel le procédé comprend en outre la détection ou la mesure du taux d'antigène carcino-embryonnaire (ACE).

FIGURE 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005019826 A
- WO 2013030579 A
- WO 2013030577 A
- WO 2013084002 A

**Non-patent literature cited in the description**

- **ALLEN et al.** *Nucleic Acids Research,* 2004, vol. 32 (3), e38
- **ESTELLER.** *Nature Reviews Genetics,* 2007, vol. 8, 286-298
- **FEINBERG ; VOGELSTEIN.** *Nature,* 1983, vol. 301, 89-92
- **GRUTZMANN et al.** *PLoS ONE,* 2008, vol. 3 (11), e3759
- **HERVOUET et al.** *PLoS ONE,* 2010, vol. 5 (6), e11333
- **HERRANZ ; ESTELLER.** *Methods Mol. Biol.,* 2007, vol. 361, 25-62
- **HOLDENRIEDER et al.** *Int. J. Cancer (Pred. Oncol.),* 2001, vol. 95, 114-120
- *The Medical Letter on Drugs and Therapeutics,* 2014, vol. 56, 100-101
- **RODRIGUEZ-PAREDES ; ESTELLER.** *Nature Medicine,* 2011, vol. 17 (3), 330-339
- **SALGAME et al.** *Nucleic Acids Research,* 1997, vol. 25 (3), 680-681
- **VAN NIEUWENHUIJZE et al.** *Ann. Rheum. Dis.,* 2003, vol. 62, 10-14